# EUROPÄISCHE PATENTSCHRIFT

(11) **EP 0 806 430 B1**
(45) Veröffentlichungstag und Bekanntmachung des Hinweises auf die Patenterteilung: **24.07.2002**
(21) Anmeldenummer: 97107522.1
(22) Anmeldetag: 07.05.1997
(51) Int. Cl.: C07H 3/04, C07B 41/08, C07H 7/033, C07C 51/235

(54) **Verfahren und Vorrichtung zur Oxidation von Kohlenhydraten, Kohlenhydratderivaten oder primären Alkoholen bei dem eine katalysierte Oxidation stattfindet.**
Process and apparatus for the oxidation of carbohydrates, carbohydrate derivatives or primary alcohols comprising a catalytic oxidation
Procédé et dispositif pour l'oxydation d'hydrates de carbone, des dérivés d'hydrates de carbone ou des alcools primaires à la base d'une oxydation catalytique

(30) Priorität: 10.05.1996 DE 19619016
(43) Veröffentlichungstag der Anmeldung: 12.11.1997
(73) Patentinhaber: SÜDZUCKER AKTIENGESELLSCHAFT MANNHEIM/OCHSENFURT, 68165 Mannheim (DE)
(72) Erfinder: Kunz, Markwart, Dr., 67550 Worms (DE); Schwarz, Andreas,, 38108 Braunschweig (DE)
(74) Vertreter: Einsel, Martin, Dipl.-Phys.

(56) Entgegenhaltungen:
- WO-A-94/20448

## Beschreibung

Die Erfindung betrifft ein Verfahren und eine Vorrichtung zur Oxidation von Kohlenhydraten, Kohlenhydratderivaten oder primären Alkoholen, bei dem eine katalysierte Oxidation stattfindet.

Derartige Oxidationsverfahren sind von hohem industriellem Interesse. Im Vergleich zu Konkurrenzverfahren, beispielsweise mikrobieller Oxidation, Elektrooxidation oder chemischer Oxidation besitzen sie bedeutsame Vorteile. Beispielsweise sind bei der mikrobiellen Fermentation unter anderem die sterile Fahrweise, die Biokatalysatorabtrennung sowie die übrige Produktaufarbeitung als aufwendig anzusehen, wenn auch die gewünschte Selektivität bei großtechnisch fermentativ hergestellten Produkten sehr hoch sein kann, während bei absatzweise betriebenen katalytischen Verfahren niedrigere Selektivitäten festgestellt wurden.

Erst durch die Entwicklung der kontinuierlichen heterogen katalytischen Oxidation etwa gemäß der WO 94/20448 konnten neben den verfahrenstechnischen Vorteilen auch die Katalysatoraktivität und -selektivität gesteigert werden, beispielsweise auf 92%.

Wünschenswert wäre es, diese Selektivität weiter zu verbessern. So könnte man daran denken, eine Dotierung der verwendeten Edelmetallkatalysatoren mit Blei oder Wismut vorzunehmen. So wird eine absatzweise betriebene Oxidation von Glucose zu Gluconsäure unter Verwendung eines Palladium/Wismut/Kohlenstoff-Katalysators und eine Erzielung einer Selektivität von über 99% selbst bei sehr hohem Umsatz von Michèle Besson at al, Catalytic Oxidation of Glucose and Bismut-Promoted Palladium Catalysts, in: Journal of Catalysis 152 (1995), 116-121 beschrieben. Bei einer testweisen Anwendung solcher Mischmetallkontakte in kontinuierlichen Verfahren ließen sich zwar ebenfalls gute Anfangsselektivitäten feststellen, diese verschlechterten sich jedoch schnell mit zunehmender Reaktionszeit. Die offenbar guten Ergebnisse der mit absatzweise betriebener Oxidation untersuchten Mischmetallkontakte sind daher auf eine kontinuierliche Reaktionsführung nicht übertragbar.

Eine weitere Möglichkeit könnte an sich in der Verwendung von Katalysatoren mit einer sehr großen aktiven Oberfläche gesehen werden. Solche Katalysatoren haben jedoch den Nachteil, daß sie neben der gewünschten Monosäureproduktion auch zu einer signifikanten Menge an höheroxidierten Nebenprodukten führen. Die größere Aktivität führt also zu einer verschlechterten Selektivität bezüglich der Monooxidationsprodukte.

Aufgabe der vorliegenden Erfindung ist es, ein Verfahren und eine Vorrichtung zur Oxidation von Kohlenhydraten, Kohlenhydratderivaten oder primären Alkoholen vorzuschlagen, das eine Verbesserung an Selektivität und/oder Aktivität ohne die beschriebenen Nachteile aufweist.

Diese Aufgabe wird dadurch gelöst, daß während der katalysierten Reaktion in der Katalysatorstufe ein elektrisches Feld angelegt wird.

Diese bisher auf diesem Gebiet unbekannte Maßnahme führt unerwartet zu der gewünschten Selektivitätserhöhung. Bei einer kontinuierlichen Monosäureproduktion wie sie etwa aus der WO 94/20448 bekannt ist, läßt sich der Anteil der höheroxidierten Nebenprodukte noch weiter als dort beschrieben verringern.

Besonders vorteilhaft kann diese Maßnahme verwendet werden, wenn gleichzeitig in der Katalysatorschüttung Trägermaterialien mit einer hochporösen Oberfläche, also einer sehr großen aktiven Oberfläche verwendet werden. Durch das Anlegen des elektrischen Feldes kann das entstehende höher oxidierte Nebenprodukt gerade bei der Verwendung derartiger Trägermaterialien deutlich verringert werden. Es kann also die größere Aktivität dieser sonst wegen der geringeren Selektivität nachteiligen Trägermaterialien mit einer höheren Selektivität kombiniert werden, was die Gesamtausbeute, gerade im Hinblick auf eine gute Raum-Zeit-Ausbeute hin verbessert.

Diese erfinderischen Gedanken harmonisieren besonders gut mit den weiteren aus der WO 94/20448 bekannten Stufen, nämlich der Zuführung der Ausgangsstoffe des Katalysators zu einer Elektrodialysestufe zur Abtrennung der oxidierten Produkte. Die nichtoxidierten Stoffe werden dann zur Oxidationsstufe und damit zum Katalysator zurückgeführt.

Vorteilhaft wird bei dem elektrischen Feld eine für elektrochemische Verfahren ungewöhnlich hohe Spannung von mehr als 10 V, bevorzugt eine Gleichspannung um etwa 30 V herum angelegt.

Bei bestimmten räumlichen Anordnungen sind aber auch andere Spannungen, auch erheblich niedrigere Spannungen um etwa 1 V einsetzbar, um den gewünschten Effekt zu erzielen. Möglich ist auch eine Hintereinanderschaltung von z.B. 20 Elektroden, wobei die Katalysatorschüttung in mehrere flache Horden dazwischen angeordnet wird.

Die Richtung der Gleichspannung, also die Anordnung von Plus- und Minuspol, ist zwar relevant, aber nicht allein entscheidend. Wird beispielsweise das Feld so angelegt, daß die Feldlinien etwa parallel zur Diffusionsrichtung der oxidierten Produkte laufen, so sollte in bestimmten Zeitabständen die Spannungsrichtung umgedreht, also Plus- und Minuspol vertauscht werden, wodurch die Selektivitätssteigerung ständig erhalten bleibt. Bei ständig in gleicher Richtung liegenden Spannung nimmt der Effekt im Zeitablauf ab.

Eine weitere Variante besteht darin, das elektrische Feld quer zur Diffusionsrichtung anzulegen. Der mikrochemische Effekt des elektrischen Feldes ist noch nicht in allen Einzelheiten geklärt. Möglicherweise führt das elektrische Feld zu einer rascheren Trennung der oxidierten Produkte von den Katalysatorelementen, was eine Mehrfachoxidation und damit Senkung der Selektivität verhindert. Dabei käme es auf die grundsätzliche Feldrichtung dann nicht an.

Im folgenden soll am Beispiel der kontinuierlichen Monooxidation von Saccharose das erfindungsgemäße Verfahren näher erläutert werden. Es zeigen:
- Fig. 1: eine schematische Darstellung des Katalysatorbereiches; und
- Fig. 2: eine Kurve mit einem Reaktionsversuchsverlauf.

Die kontinuierliche katalytische Oxidation von Saccharose liefert drei Monooxidationsprodukte:

| | |
|---|---|
| C₆-Säure | 2-O-(1-O-α-D-Glucopyranosyl)-β-D-fructofuranuronsäure |
| C_{6'}-Säure | 1-O-(2-O-β-D-Fructofurannosyl)-α-D-glucopyranuronid |
| C₁-Säure | 2-O-(1-O-α-D-Glucopyranosyl)-β-D-arabino-2-hexulofuranonsäure |

Als Hauptnebenprodukt der gezielten Monooxidation kann die

| | |
|---|---|
| C_{66'}-Disäure | 1-O-(2-O-β-D-Fructofuranuronyl)-α-D-glucopyranuronid |

sowohl qualitativ als auch quantitativ HPLC-analytisch erfaßt werden.

Die Monooxidation der Saccharose erfolgt prinzipiell so, wie aus WO 94/20448 her bekannt auf deren Offenbarungsgehalt auch insgesamt Bezug genommen wird: Eine wäßrige Saccharoselösung wird in einem Gefäß durch einen Frittenboden mit einem sauerstoffhaltigen Gas begast; mittels einer pH-Regelung wird der pH-Wert durch Zudosierung von Neutralisationsmittel auf dem eingestellten Sollwert gehalten. Die so mit Sauerstoff angereicherte thermostatisierte Saccharoselösung wird mit einer Pumpe einem Oxidationsreaktor 10 zugeführt (bei 11), der neben einer Katalysatorschüttung 20 nun Elektroden 31, 32 enthält, mit deren Hilfe an die Schüttung 20 ein elektrisches Feld 30 angelegt wird.

Bei 12 verlassen die Reaktionsprodukte den Oxidationsreaktor 10. Nach der Oxidationsstufe erfolgt mittels einer (nicht dargestellten) Elektrodialyseeinheit die Abtrennung der Oxidationsprodukte. Das nichtoxidierte Edukt wird anschließend in das erste Gefäß zurückgeführt, wo Luftbegasung, pH-Regulierung und Saccharosenachdosierung stattfinden. Danach setzt sich bei 11 der Kreisprozeß fort.

Durch Anlegen des elektrischen Feldes 30 an die Katalysatorschüttung 20 werden die gebildeten deprotonierten Carbonsäureanionen beschleunigt aus der Katalysatorschüttung 20 herausgezogen, d.h., vor allem bei denjenigen Katalysatoren, wo der Abtransport der Reaktionsprodukte von den katalytisch aktiven Zentren weg limitierend auf die beobachtbare Gesamtgeschwindigkeit wirkt, kann dieser Schritt beschleunigt werden. Dadurch können sich die scheinbare Katalsysatorenaktivität sowie die Selektivität der Reaktion ändern.

Überraschend bei dieser Erfindung war die Tatsache, daß auch durch das Anlegen hoher Spannungen - bis 30 V - an die Elektroden 31, 32 keine sekundär ablaufenden elektrochemischen Reaktionen zu beobachten waren. Weder konnte an den Elektroden eine Bläschenentwicklung entdeckt werden, noch änderte sich die Selektivität der katalytischen Oxidation nachteilig, d.h., der Anteil an höheroxidierten Produkten nahm ab statt zu. Lediglich bei höheren ph-Werten ließ sich parallel zu einer bemerkenswerten Geschwindigkeitssteigerung eine Wasserstoffentwicklung messen, so daß eine mögliche Elektrooxidation der Kohlenhydrate nicht generell ausgeschlossen werden kann. Aber auch unter diesen Bedingungen konnte keine Verschlechterung bzw. signifikante Veränderung der Selektivität der Oxidation festgestellt werden. Ebensowenig konnten kettenverkürzte Kohlenhydratderivate nachgewiesen werden, wobei als Elektrodenmaterialien goldbeschichtetes Stahldrahtnetz sowie beschichtete Platinnetzelektroden untersucht wurden.

Die untersuchten Katalysatoren sind kommerziell erhältliche Pt/C-Trägerkatalysatoren mit unterschiedlichem Platingehalt und unterschiedlicher Porosität der Aktivkohle. Bei den Katalysatoren wurde, um sie im Festbett einsetzen zu können, entweder der Feinanteil durch Klassieren entfernt oder sie wurden in geeigneten Matrices wie Polyvinylalkohol immobilisiert.

Bei der kontinuierlichen Monooxidation der Saccharose mit einem Pt/C-Trägerkatalysator mit vergleichsweise geringer Porosität waren beim Anlegen einer Gleichspannung an die Katalysatorschüttung die selektivitätsverändernden oder aktivitätssteigernden Effekte relativ gering.

Nimmt man stattdessen zur Saccharosemonooxidation einen Pt/C-Trägerkatalysator mit relativ hoher Porosität, so lassen sich jedoch Selektivität und Aktivität des Katalysators durch Anlegen einer Gleichspannung an die Katalysatorschüttung deutlich verändern. Nach einigen Tagen verschlechterte sich die Katalysatoraktivität kontinuierlich, was eventuell mit einer dauerhaften Polarisierung der Elektroden oder der Katalysatorschüttung zu erklären ist.

Durch periodisches Vertauschen von Plus- und Minuspol des Kondensators (also Umkehrung des elektrischen Feldes 30) ließen sich Selektivität und scheinbare Aktivität des verwendeten Katalysators dann dauerhaft verändern. Die periodische Umpolung geschah in Versuchen mit Hilfe eines Relais, das mit einem Netzgerät umgeschaltet wurde, welches wiederum durch eine Tageszeitschaltuhr ein- oder ausgestellt wurde.

Diese hochporösen Pt/C-Trägerkatalysatoren sind in ihrer scheinbaren Aktivität auch durch die Elektrodialysespannung beeinflußbar. So kann man durch Wahl einer sehr hohen Elektrodialysespannung zwar ebenfalls hohe scheinbare Katalysatoraktivität erzeugen, jedoch steigt unter diesen Bedingungen die Salzfracht und der pH-Wert im Konzentratkreislauf der Elektrodialyse extrem an, d.h., das zur Neutralisation der Oxidationsprodukte automatisch zugegebene Titrationsmittel wird teilweise durch die hohe Elektrodialysespannung mit in den Konzentratkreislauf gezogen. Das erhöht die Produktaufarbeitungskosten und ist daher sehr nachteilig. Unter solchen Betriebsbedingungen kann auch durch Anlegen einer Gleichspannung an die Katalysatorschüttung nicht immer eine zusätzliche Reaktionsbeschleunigung erreicht werden, sondern lediglich eine Selektivitätsveränderung.

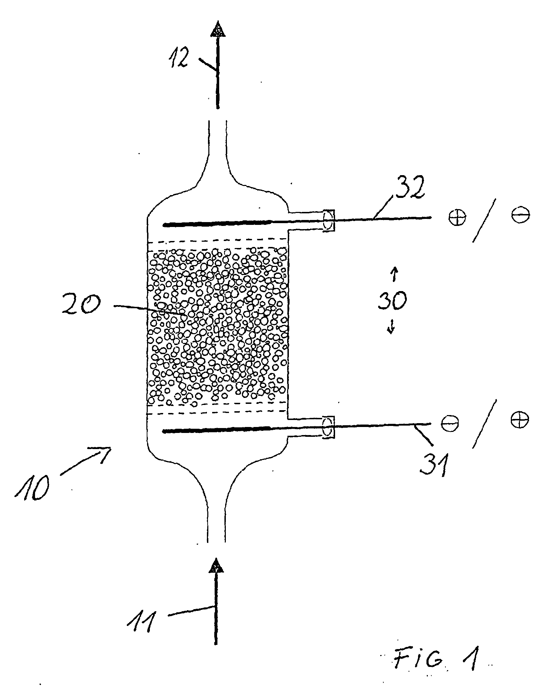

In der **Figur 1** ist ein Aufbau mit einem elektrischen Feld 30 parallel zur Diffusionsrichtung der Oxidationsprodukte wiedergegeben. Dies hat sich als nicht zwingend herausgestellt. Ebenso ist es auch möglich, die Elektroden 31 und/oder 32 in Kontakt mit der Katalysatorschüttung 20 oder auch ohne Kontakt zum Fluid bzw. zur Katalysatorschüttung anzuordnen. Der Elektrodenabstand ist dabei in allen Fällen variierbar, wobei mit zunehmender Verringerung des Elektrodenabstandes eine Effektverstärkung zu erwarten ist.

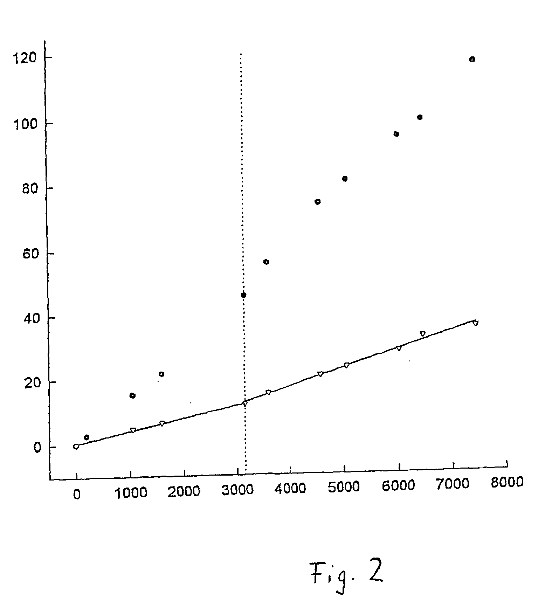

In einem typischen Versuch (vgl. Figur 2) wird Saccharose zunächst, um eine Charakterisierung des verwendeten Katalysators zu ermöglichen, einige Tage lang monooxidiert, ohne daß an die Katalysatorschüttung ein elektrisches Feld angelegt wird. Dabei wird die Elektrodialysespannung etwas höher eingestellt, als für ein an anorganischen Salzen weitgehend armes Konzentrat erforderlich ist, damit nach Anlegen des elektrischen Feldes an die Katalysatorschüttung die vermehrt gebildeten oxidierten Reaktionsprodukte ebenfalls durch die Elektrodialyse aus dem Oxidationskreislauf entfernt werden. Somit wird ohne eine zweite Parameterveränderung sichergestellt, daß die stationäre Produktkonzentration im Oxidationskreislauf sich nur unwesentlich ändert und somit vergleichbare Bedingungen vorliegen. Das Produktgemisch bestand typischerweise aus 46.6% C₆-Säure, 43.4% C_{6'}-Säure, 3.7% C₁-Säure und 4.4% C_{66'}-Disäure, wobei letztere als Hauptnebenprodukt anzusehen ist. Die Selektivität der Saccharosemonosäurebildung betrug demnach 93.6%.

Durch das Anlegen eines elektrischen Feldes an die Katalysatorschüttung ließ sich die scheinbare Aktivität um den Faktor 1.5 steigern, die Zusammensetzung des so erhaltenen Produktgemisches änderte sich wie folgt: 64.4% C₆-Säure, 23.4% C_{6'}-Säure, 7.0% C₁-Säure und 3.2% C_{66'}-Disäure. Neben der scheinbaren Aktivitätsverbesserung ließ sich auch die Selektivität der Saccharosemonosäurebildung auf 95.8% steigern, ferner wurde das Verhältnis der gebildeten Monosäuren in signifikanter Weise verändert. Als zusätzlicher Verfahrensvorteil fiel auf, daß sich der Verbrauch an Titrationsmittel trotz der scheinbaren Aktivitätssteigerung des Katalysators nicht erhöhte, d.h., die Belastung des Produktgemisches mit anorganischen Salzen verringert sich.

**Figur 2** zeigt diesen Verfahrensablauf. Auf der Ordinate ist die Reaktionszeit in Minuten aufgetragen, auf der Abszisse der Verbrauch in 1 M K₂CO₃ in Millilitern (der Kurvenverlauf ist mit ausgefüllten Punkten eingezeichnet) und die Produktanreicherung im Konzentrat in Millimol (dieser Kurvenverlauf ist in nicht ausgefüllten Dreiecken eingezeichnet.

Nach etwa 3100 Minuten Reaktionszeit wurde hier das elektrische Feld angelegt (senkrechte punktierte Linie).

Ebenfalls wurde experimentell mit Hilfe eines dreißigtägigen Langzeitversuches sichergestellt, daß die durch das elektrische Feld verursachten Effekte nicht bloß kurzzeitiger Natur sind.

Die angeführten Beispiele belegen, daß sich Aktivität und Selektivität bei bestimmten Katalysatortypen durch ein elektrisches Feld langfristig günstig beeinflussen bzw. verändern lassen. Überraschend war allerdings, daß nach Abschalten des elektrischen Feldes die Katalysatorselektivität teilweise modifiziert blieb, d.h., erst durch Regenerierung des Katalysators z.B. durch längeres Begasen mit H₂ ließen sich Anfangsselektivitäten und scheinbare Aktivität wiederherstellen.

### Beispiel 1:

### Kontinuierliche Monooxidation von Saccharose

In der aus der WO 94/20448 bekannten Oxidationsapparatur wird der Oxidationsreaktor so modifiziert, daß sich oberhalb und unterhalb der Katalysatorschüttung (29 g Katalysatortrockenmasse Typ F 105 XR/W 1% PT/C, Fa. Degussa, immobilisiert in Polyvinylalkohol: Beladung der PVAL-Partikel mit Katalysatortrockenmasse 12%, mittlerer Durchmesser des Immobilisats: 3 mm; anschließend wurde das Immobilisat 12 h bei 50° C getrocknet) je eine Elektrode befindet. Der Elektrodenabstand beträgt ca 10 cm, Elektrodenmaterial ist goldbeschichtetes Stahldrahtnetz, der Elektrodendurchmesser beträgt 4,5 cm. Mit diesem modifizierten Oxidationsreaktor wird - zunächst ohne daß an die Elektroden eine Spannung angelegt wird - eine wäßrige 0.15 molare Saccharoselösung bei einem pH-Wert von 6.5 und einer Temperatur von 35° C oxidiert. Der pH-Wert wird durchTitration der entstandenen Säuren mit 1 M K₂CO₃ auf dem Sollwert gehalten. Die Elektrodialyse ist mit 6 AMV/CMV Membranpaaren (Fa. Berghof) ausgestattet (entsprechend 360 cm² effektiver Membranfläche) und wird bei einer Spannung von 2.0 V betrieben. Begast wird mit synthetischer Luft; die Begasungsraten sind 100 ml O₂/min und 400 ml N₂/min.

Die beobachtete Katalysatoraktivität wird HPLC-analytisch aus der Summe der pro Zeiteinheit im Konzentratkreislauf sich anreichernden Oxidationsprodukte bestimmt und auf 1 g Pt normiert. Die Produktzusammensetzung wird ebenfalls HPLC-analytisch durch Konzentratuntersuchungen bestimmt.

Nach ca. 2 - 3 Tagen wurde an die Elektroden eine Gleichspannung von 10 V angelegt, wobei mittels einer Relaisschaltung alle 90 min Plus- und Minuspol vertauscht wurden. Vor und nach Anlegen des elektrischen Feldes an die Katalysatorschüttung ergaben sich jeweils für die stationären Betriebszustände der Oxidationsanlage für scheinbare Aktivität und Selektivität bezüglich der Hauptprodukte die folgenden Werte:

| Kondensatorfeld | Katalysatoraktivität [mmol/min*1g Pt] | Produktzusammensetzung [%] | | | |
|---|---|---|---|---|---|
| | | C₆ | C_{6'} | C₁ | C_{66'}-Di |
| - | 3.7 * 10⁻³ | 46.6 | 43.4 | 3.7 | 4.4 |
| 10 V / alle 90 min Umpolung | 5.4 * 10⁻³ | 64.4 | 23.4 | 7.0 | 3.2 |

### Beispiel 2

Analog zu Beispiel 1 wurde Saccharose zunächst ca. 5 Tage lang monooxidiert, ohne daß an die Katalysatorschüttung ein elektrisches Feld angelegt wurde. Als Katalysator wurde ebenfalls ein Pt/C-Trägerkatalysator getestet (Typ F 105; R/W 5% Pt/C, Fa. Degussa, Immobilisierung wie bei Beispiel 1, jedoch ohne thermische Nachbehandlung des Immobilisats, immobiliserte Pt-Menge: 0.925 g). Die gewählte Elektrodialysespannung betrug 4.0 V. Nach der Charakterisierung des stationären Betriebszustandes wurde an die Elektroden (Material: beschichtetes Pt-Netz, Elektrodenabstand: 27 cm) eine elektrische Gleichspannung von 30 V angelegt, wobei alle 45 min. eine Umpolung erfolgte. Durch das elektrische Feld änderten sich die Katalysatorkenndaten wie folgt:

| Kondensatorfeld | Katalysatoraktivität [mmol/min*1g Pt] | Produktzusammensetzung [%] | | | |
|---|---|---|---|---|---|
| | | C₆ | C_{6'} | C₁ | C_{66'}-Di |
| - | 3.8 * 10⁻³ | 44.5 | 43.0 | 5.4 | 5.1 |
| 30 V / alle 45 min Umpolung | 3.4 * 10⁻³ | 49.9 | 33.3 | 10.1 | 4.7 |

### Beispiel 3

Durch Erhöhung der Elektrodialysespannung von 4.0 auf 5.0 V konnte die Produktzusammensetzung im Vergleich zu Beispiel 2 noch weiter in Richtung Monooxidationsprodukte verschoben werden:
52.7% C₆-Säure, 32.8% C_{6'}-Säure, 8.3% C₁-Säure und 4.1% C_{66'}-Disäure.

## Patentansprüche

1. Verfahren Oxidation von Kohlenhydraten, Kohlenhydratderivaten oder primären Alkoholen, bei dem eine katalysierte Oxidation unter Verwendung von Sauerstoff oder sauerstoffhaltigen Gasen als Oxidationsmittel und unter Gebrauch von Metallkatalysatoren stattfindet, und bei dem eine Elektrodialyse eingesetzt wird,
**dadurch gekennzeichnet,**
**dass** während der katalysierten Reaktion in der Katalysatorstufe ein elektrisches Feld angelegt wird.

2. Verfahren nach Anspruch 1,
**dadurch gekennzeichnet,**
**dass** die katalysierte Oxidation an einer Katalysatorschüttung mit Edelmetallen der 8. Nebengruppe und/oder mit einer Dotierung insbesondere mit Blei oder Wismut stattfindet.

3. Verfahren nach Anspruch 1 oder 2,
**dadurch gekennzeichnet,**
**dass** die katalytische Oxidation an einer Katalysatorschüttung mit hochporösen Trägermaterialien stattfindet.

4. Verfahren nach einem der vorstehenden Ansprüche
**dadurch gekennzeichnet,**
**dass** das elektrische Feld quer zur Diffusionsrichtung der zu oxidierenden Stoffe angelegt wird.

5. Verfahren nach einem der Ansprüche 1 bis 3
**dadurch gekennzeichnet,**
**dass** das elektrische Feld parallel zur Diffusionsrichtung der zu oxidierenden Stoffe angelegt und seine Feldrichtung in Zeitabständen umgekehrt wird.

6. Verfahren nach einem der vorstehenden Ansprüche,
**dadurch gekennzeichnet,**
**dass** als Spannung eine elektrische Gleichspannung von mehr als 10 V, insbesondere um 30 V angelegt wird.

7. Vorrichtung zur Oxidation von Kohlenhydraten, Kohlenhydratderivaten oder primären Alkoholen mit einem Verfahren nach einem der vorstehenden Ansprüche, wobei die Vorrichtung einen Reaktionsraum besitzt, der den Katalysator enthält, sowie Zu- und Ableitungen für die Produktströme und eine Elektrodialyseeinrichtung aufweist
**dadurch gekennzeichnet,**
**dass** die Katalysatorschüttung (20) in einem elektrischen Feld (30) mit Elektroden angeordnet ist.

8. Vorrichtung nach Anspruch 7,
**dadurch gekennzeichnet,**
**dass** das elektrische Feld (30) durch mindestens zwei Elektroden (31, 32) aufgebaut wird und dass die Elektroden (31, 32) im Kontakt mit der Katalysatorschüttung (20) und dem Fluid aus den zu oxidierenden Stoffen angeordnet sind.

9. Vorrichtung nach Anspruch 7,
**dadurch gekennzeichnet,**
**dass** das elektrische Feld (30) durch mindestens zwei Elektroden (31, 32) aufgebaut wird und dass die Elektroden (31, 32) im Kontakt mit Fluid aus den zu oxidierenden Stoffen, aber ohne Kontakt mit der Katalysatorschüttung (20), angeordnet sind.

10. Vorrichtung nach Anspruch 9,
**dadurch gekennzeichnet,**
**dass** die Katalysatorschüttung (20) zwischen zwei Frittenböden angeordnet ist und sich die Elektroden (31, 32) außerhalb der Katalysatorschüttung (20) und der beiden Frittenböden befinden, wobei das elektrische Feld (30) parallel oder senkrecht zu den Frittenböden angelegt ist.

11. Vorrichtung nach Anspruch 7,
**dadurch gekennzeichnet,**
**dass** das elektrische Feld (30) durch, mindestens zwei Elektroden (31, 32) aufgebaut wird und dass die Elektroden ohne Kontakt zum Fluid aus den zu oxidierenden Stoffen und ohne Kontakt zur Katalysatorschüttung (20) angeordnet sind.

12. Vorrichtung nach einem der Ansprüche 7 bis 11,
**dadurch gekennzeichnet,**
**dass** die Katalysatorschüttung (20) Katalysatoren mit hochporösen Trägermaterialien aufweist.

## Claims

1. Process for the oxidation of carbohydrates, carbohydrate derivatives or primary alcohols, in which a catalysed oxidation takes place with the use of oxygen or oxygen- containing gases as oxidising agent and using metal catalysts, and in which an electrodialysis is employed,
**characterised in that**
during the catalysed reaction an electric field is applied in the catalyst stage.

2. Process according to claim 1,
**characterised in that**
the catalysed oxidation takes place on a catalyst bed with precious metals of the 8th sub-group and/or with a doping in particular with lead or bismuth.

3. Process according to claim 1 or 2,
**characterised in that**
the catalytic oxidation takes place on a catalytic bed with highly porous support materials.

4. Process according to any one of the preceding claims,
**characterised in that**
the electric field is applied transversely of the diffusion direction of the substances to be oxidised.

5. Process according to any one of claims 1 to 3,
**characterised in that**
the electric field is applied parallel with the diffusion direction of the substances to be oxidised and its field direction is reversed at intervals of time.

6. Process according to any one of the preceding claims,
**characterised in that**
an electric d.c. voltage of more than 10 V, in particular of about 30 V, is applied as voltage.

7. Apparatus for the oxidation of carbohydrates, carbohydrate derivatives or primary alcohols by a process according to any one of the preceding claims, wherein the apparatus possesses a reaction chamber which contains the catalyst, together with feed and discharge lines for the product streams and an electrodialysis unit,
**characterised in that**
the catalyst bed (20) is arranged in an electric field (30) with electrodes.

8. Apparatus according to claim 7.
**characterised in that**
that the electric field (30) is formed by at least two electrodes (31, 32) and that the electrodes (31, 32) are arranged in contact with the catalyst bed (20) and the fluid from the substances to be oxidised.

9. Apparatus according to claim 7,
**characterised in that**
the electric field (30) is formed by at least two electrodes (31,32) and that the electrodes (31, 32) are arranged in contact with fluid from the substances to be oxidised, but without contact with the catalyst bed (20).

10. Apparatus according to claim 9,
**characterised in that**
the catalyst bed (20) is arranged between two fritted bottoms and the electrodes (31,32) are located outside the catalyst bed (20) and the two fritted bottoms, wherein the electric field (30) is applied parallel with or perpendicular to the fritted bottoms.

11. Apparatus according to claim 7
**characterised in that**
the electric field (30) is formed by at least two electrodes (31,32) and that the electrodes are arranged without contact with the fluid from the substances to be oxidised and without contact with the catalyst bed (20).

12. Apparatus according to any one of claims 7 to 11,
**characterised in that**
the catalyst bed (20) comprises catalysts with highly porous support materials.

## Revendications

1. Procédé d'oxydation de glucides, de dérivés de glucides ou d'alcools primaires, lors duquel a lieu une oxydation catalysée par utilisation d'oxygène ou de gaz contenant de l'oxygène en tant qu'agents d'oxydation et avec utilisation de catalyseurs métalliques, et lors duquel une électrodialyse est utilisée, **caractérisé en ce qu'**un champ électrique est appliqué pendant la réaction catalysée dans l'étape à catalyseur.

2. Procédé selon la revendication 1, **caractérisé en ce que** l'oxydation catalysée a lieu sur un empilement de catalyseur avec des métaux du 8èmè groupe secondaire et/ou avec un agent de dopage, en particulier avec du plomb ou du bismuth.

3. Procédé selon la revendication 1 ou 2, **caractérisé en ce que** l'oxydation catalytique a lieu sur un empilement de catalyseur avec des matériaux support fortement poreux.

4. Procédé selon l'une quelconque des revendications précédentes, **caractérisé en ce que** le champ électrique est appliqué perpendiculairement à la direction de diffusion des substances à oxyder.

5. Procédé selon l'une quelconque des revendications 1 à 3, **caractérisé en ce que** le champ électrique est appliqué parallèlement à la direction de diffusion de la substance à oxyder et **en ce que** sa direction de champ est inversée à des intervalles de temps.

6. Procédé selon l'une quelconque des revendications précédentes, **caractérisé en ce que** l'on applique, en tant que tension, une tension électrique continue de plus de 10 V, en particulier se situant aux alentours de 30 V.

7. Dispositif d'oxydation de glucides, de dérivés de glucides ou d'alcools primaires à l'aide d'un procédé selon l'une quelconque des revendications précédentes, le dispositif possédant un espace de réaction, qui contient le catalyseur, et qui présente des conduits d'amenée et d'évacuation pour les courants de produit et un équipement d'électrodialyse, **caractérisé en ce que** l'empilement du catalyseur (20) est disposé dans un champ électrique (30) avec des électrodes.

8. Dispositif selon la revendication 7, **caractérisé en ce que** le champ électrique (30) est constitué par au moins deux électrodes (31, 32) et **en ce que** les électrodes (31, 32) sont disposées en contact avec l'empilement de catalyseur (20) et le fluide en provenance des substances à oxyder.

9. Dispositif selon la revendication 7, **caractérisé en ce que** le champ électrique (30) est constitué par au moins deux électrodes (31, 32) et **en ce que** les électrodes sont disposées en contact avec le fluide en provenance des substances à oxyder, mais sans contact avec l'empilement de catalyseur (20).

10. Dispositif selon la revendication 9, **caractérisé en ce que** l'empilement de catalyseur (20) est disposé entre deux fonds de fritte et **en ce que** les électrodes (31, 32) se trouvent à l'extérieur de l'empilement de catalyseur (20) et des deux fonds de fritte, le champ électrique (30) étant disposé parallèlement ou perpendiculairement aux fonds de fritte.

11. Dispositif selon la revendication 7, **caractérisé en ce que** le champ électrique (30) est constitué par au moins deux électrodes (31, 32) et **en ce que** les électrodes sont disposées sans contact avec le fluide en provenance des substances à oxyder et sans contact avec l'empilement de catalyseur (20).

12. Dispositif selon l'une quelconque des revendications 7 à 11, **caractérisé en ce que** l'empilement de catalyseur (20) présente des matériaux support fortement poreux.
